(19) Europäisches Patentamt
European Patent Office
Office européen des brevets

(11) Publication number: 0 260 042 B1

(12) **EUROPEAN PATENT SPECIFICATION**

(45) Date of publication of patent specification: 27.12.91

(51) Int. Cl.[5]: C07D 499/00, A61K 31/43, //C07D335/02,C07D333/48, C07F7/18

(21) Application number: 87307636.8

(22) Date of filing: 28.08.87

The file contains technical information submitted after the application was filed and not included in this specification

(54) **2-(1-Oxo-1-imino-1-thiacycloalkyl)-Thiopenem derivatives.**

(30) Priority: 04.09.86 PCT/US86/01813

(43) Date of publication of application:
16.03.88 Bulletin 88/11

(45) Publication of the grant of the patent:
27.12.91 Bulletin 91/52

(84) Designated Contracting States:
AT BE CH DE ES FR GB GR IT LI LU NL SE

(56) References cited:
EP-A- 0 130 025

ANGEW. CHEM. INT. ED. ENGL., (1985), 24, page 180

J. MED. CHEM., (1983), 26, page 259

(73) Proprietor: PFIZER INC.
235 East 42nd Street
New York, N.Y. 10017(US)

(72) Inventor: Hamanaka, Ernest Seiichi
40, Eagle Ridge Road
Gales Ferry Connecticut(US)

(74) Representative: Wilkinson, Stephen John et al
Stevens, Hewlett & Perkins 1 St. Augustine's Place
Bristol BS1 4UD(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 260 042 B1

## Description

The present invention relates to novel antibacterial agents of the B-lactam type. Chemically, the antibacterial agents of the present invention are classified as 6-(1-hydroxyethyl)-2-substituted-pen-2-em-3-carboxylic acids and the pharmaceutically acceptable salts and esters thereof.

2-Substituted-2-penem-3-carboxylic acid compounds have been disclosed in U.S. Patent Nos. 4,155,912, 4,260,618, 4,272,437, 4,395,418, 4,595,539, Belgian Patent No. 866,845, published European Patent Application Nos. 636 and 2,210 and Journal of the American Chemical Society 101, 2210 (1979). According to the abstract thereof published by Derwent Publications Ltd., published Japanese Patent Application No. 66694/1979 also discloses 2-substituted-2-penem-3-carboxylic acid compounds.

EP-A-0130025 discloses some 6-alpha-hydroxyethyl-2-substituted-2-penem-3-carboxylic acid compounds having antibiotic activity. Examples of compounds disclosed include some containing a 2-(1,1-dioxo-(4,5 or 6-membered) thiacycloalkane)thio group.

Although B-lactam antibiotics are known, there is a continuing need for new antibiotics since continued wide scale usage selectively gives rise to resistant strains of pathogens. In addition, the known antibiotics suffer from the disadvantages of being effective against only certain types of pathogens. Accordingly, the search for new and effective antibiotics continues.

The present invention is directed to compounds of the formula

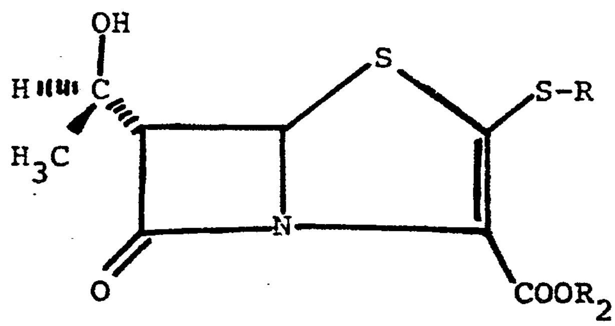

(I)

or a pharmaceutically acceptable salt thereof wherein R is a 4, 5 or 6 membered thiacycloalkane moiety of the formula

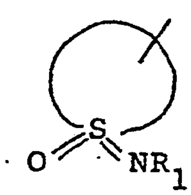

wherein $R_1$ is hydrogen, $C_1$ to $C_4$ alkoxycarbonyl, $C_1$ to $C_4$ alkylcarbonyl, $C_1$ to $C_4$ sulfonylalkyl, $C_1$ to $C_4$ alkylaminocarbonyl, and $R_2$ is hydrogen or an ester-forming group hydrolyzable in vivo.

Particularly preferred compounds include those wherein R is 1-oxo-1-imino-1-thiacyclohex-4-yl, 1-oxo-1-imino-1-thiacyclopent-3-yl, 1-oxo-1-acetylimino-1-thiacyclohex-4-yl, 1-oxo-1-acetylimino-1-thiacyclopent-3-yl, 1-oxo-1-methoxycarbonylimino-1-thiacyclohex-4-yl, 1-oxo-1-methoxycarbonylimino-1-thiacyclopent-3-yl, 1-oxo-1-methylsulfonylimino-1-thiacyclohex-4-yl, 1-oxo-1-methylsulfonylimino-1-thiacyclopent-3-yl, 1-oxo-1-methylaminocarbonylimino-1-thiacyclohex-4-yl, 1-oxo-1-methylaminocarbonylimino-1-thiacyclopent-3-yl.

Preferred compounds of the above series are either the cis or trans isomers especially those wherein R is

cis-1-oxo-1-imino-1-thiacyclopent 3-yl,
trans-1-oxo-1-imino-1-thiacyclopent-3-yl,
cis-1-oxo-1-imino-1-thiacyclohex-4-yl,
trans-1-oxo-1-imino-1-thiacyclohex-4-yl,
cis-1-oxo-1-acetylimino-1-thiacyclopent-3-yl,
trans-1-oxo-1-acetylimino-1-thiacyclopent-3-yl,

cis-1-oxo-1-acetylimino-1-thiacyclohex-4-yl,
trans-1-oxo-1-acetylimino-1-thiacyclohex-4-yl,
cis-1-oxo-1-methoxycarbonylimino-1-thiacyclopent-3-yl,
trans-1-oxo-1-methoxycarbonylimino-1-thiacyclopent-3-yl,
cis-1-oxo-1-methoxycarbonylimino-1-thiacyclohex-4-yl
trans-1-oxo-1-methoxycarbonylimino-1-thiacyclohex-4-yl,
cis-1-oxo-1-methylsulfonylimino-1-thiacyclopent-3-yl,
trans-1-oxo-1-methylsulfonylimino-1-thiacyclopent-3-yl,
cis-1-oxo-1-methylsulfonylimino-1-thiacyclohex-4-yl,
trans-1-oxo-1-methylsulfonylimino-1-thiacyclohex-4-yl,
cis-1-oxo-1-methylaminocarbonylimino-1-thiacyclopent-3-yl,
trans-1-oxo-1-methylaminocarbonylimino-1-thiacyclopent-3-yl,
cis-1-oxo-1-methylaminocarbonylimino-1-thiacyclohex-4-yl,
trans-1-oxo-1-methylaminocarbonylimino-1-thiacyclohex-4-yl.

An especially preferred group of compounds are those wherein $R_2$ is hydrogen and R is cis or trans-1-oxo-1-imino-1-thiacyclopent-3-yl or cis or trans-1-oxo-1-imino-1-thiacyclohex-4-yl.

The present invention is also directed to a pharmaceutical composition comprising a compound of Formula I and a pharmaceutically acceptable carrier or diluent and a method of treating a bacterial infection in a mammal comprising administering an antibacterially effective amount of a compound of Formula I.

In the above formulae, the schematic conforms to the accepted convention for indicating stereoisomers namely ",,,," to indicate an atom projecting into the plane of the paper (α-orientation) "▲" to indicate an atom projecting out from the plane of the paper (B orientation) and hence the plane of the molecule itself.

In the nomenclature of the R groups, the designations "cis" and "trans" refer to the relative stereochemistry of the sulfoxide oxygen and the sulfur atom of (S-R).

The compounds of the present invention are conveniently prepared from the known bicyclic nucleus of Formula II.

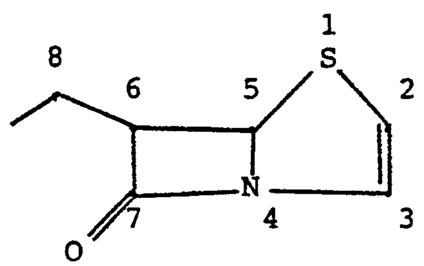

(II)

wherein the atoms are numbered as shown.

The present invention also embraces all stereoisomers of the compounds. However, it is well known in the bicyclic B-lactam antibiotics art that certain isomers of a given species are more active than the corresponding enantiomorphs. Since an asymmetric carbon atom is present at positions 5, 6 and 8, the compounds of formula I may exist in the form of racemic mixtures or as the individual dextrorotatory and levorotatory optical isomers. While the present invention includes both the racemic mixtures and resolved isomers, the preferred compounds have the 5R, 6S, 8R configuration since these have been found to possess the high antibacterial activity attributed to the racemic mixtures.

Hereinafter, the basic nucleus shown in Formula II is referred to as a "2-penem" and the ring atoms are numbered as shown.

The present invention is directed to penems substituted at the 2-position by a moiety of the formula S-R, wherein R is a 4, 5 or 6-membered thiacycloalkane moiety of the formula

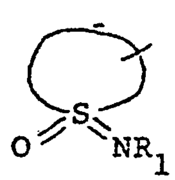

wherein $R_1$ is hydrogen, $C_1$ to $C_4$ alkoxycarbonyl, $C_1$ to $C_4$ alkylcarbonyl, $C_1$ to $C_4$ alkylsulfonyl, and $C_1$ to

$C_4$ alkylaminocarbonyl.

Exemplary groups represented by $R_1$ include:

H;

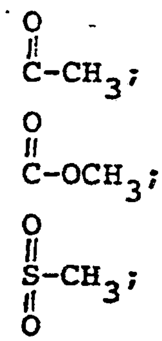

and

$CH_3NHCO$.

Preferred compounds include those wherein R is
1-oxo-1-imino-1-thiacyclohexane,
1-oxo-1-imino-1-thiacyclopentane,
1-oxo-1-imino-1-thiacyclobutane, 1-oxo-1-acetyl imino-1-thiacyclohexane,
1-oxo-1-acetylimino-1-thiacyclopentane,
1-oxo-1-acetylimino-1-thiacyclobutane,
1-oxo-1-methoxycarbonylimino-1-thiacyclohexane,
1-oxo-1-methoxycarbonylimino-1-thiacyclopentane,
1-oxo-1-methoxycarbonylimino-1-thiacyclobutane,
1-oxo-1-methylsulfonylimino-1-thiacyclohexane,
1-oxo-l-methylsulfonylimino-1-thiacyclopentane,
1-oxo-1-methylsulfonylimino-1-thiacyclobutane,
1-oxo-1-methylaminocarbonylimino-1-thiacyclohexane,
1-oxo-1-methylaminocarbonylimino-1-thiacyclopentane,
and 1-oxo-1-methylaminocarbonylimino-1-thiacyclobutane.

The present invention includes those penems wherein the 3-carboxyl group is esterified with a non-toxic ester group readily hydrolyzable in vivo. Examples of preferred ester moieties include those wherein $R_2$ is benzyl, p-nitrobenzyl (PNB), o-nitrobenzyl, t-butyl, t-butyl-dimethylsilyl, trimethylsilyl, trichloroethyl, allyl, methallyl and 2-chloroallyl. $R_2$ may also represent pharmaceutically acceptable ester moieties hydrolyzed in vivo. Typical examples of such readily hydrolyzable ester-forming moieties are alkanoyloxymethyl having from 3-8 carbon atoms, 1-(alkanoyloxy)ethyl having from 4-9 carbon atoms, 1-methyl-1-(alkanoyloxy)ethyl having from 5-10 carbon atoms, alkoxycarbonyloxymethyl having from 3-6 carbon atoms, 1-(alkoxycarbonyloxy)ethyl having from 4-7 carbon atoms, 1-methyl-1-(alkoxycarbonyloxy)ethyl having from 5-8 carbon atoms, N-(alkoxycarbonyl)aminomethyl having from 3-9 carbon atoms, 1-(N-[alkoxycarbonyl]-amino)ethyl having from 4-10 carbon atoms, 3-phthalidyl, 4-crotonolactonyl, gamma-butyrolacton-4-yl, carboxyalkylcarbonyloxymethyl having from 4-12 carbon atoms and 5-methyl-2-oxo-1,3-dioxolen-4-ylmethyl.

To prepare compounds of formula I wherein $R_2$ is a group which forms an ester which is hydrolyzed in vivo, the acid of formula I ($R_2$ is hydrogen) is reacted with a base to form the corresponding anion. Suitable cations include sodium, potassium, calcium, tetra-alkylammonium and the like. The anion can be prepared by lyophilizing an aqueous solution of I, for example, an aqueous solution containing tetrahydrofuran, and sodium bicarbonate or tetrabutyl ammonium hydroxide.

The resulting anion of I is reacted with the corresponding chloride or bromide of $R_2$ in a reaction inert solvent such as acetone or dimethylformamide at about 20° to about 50° C, preferably 25° C.

The compounds of Formula I can be conveniently synthesized according to the following schemes:

Scheme A

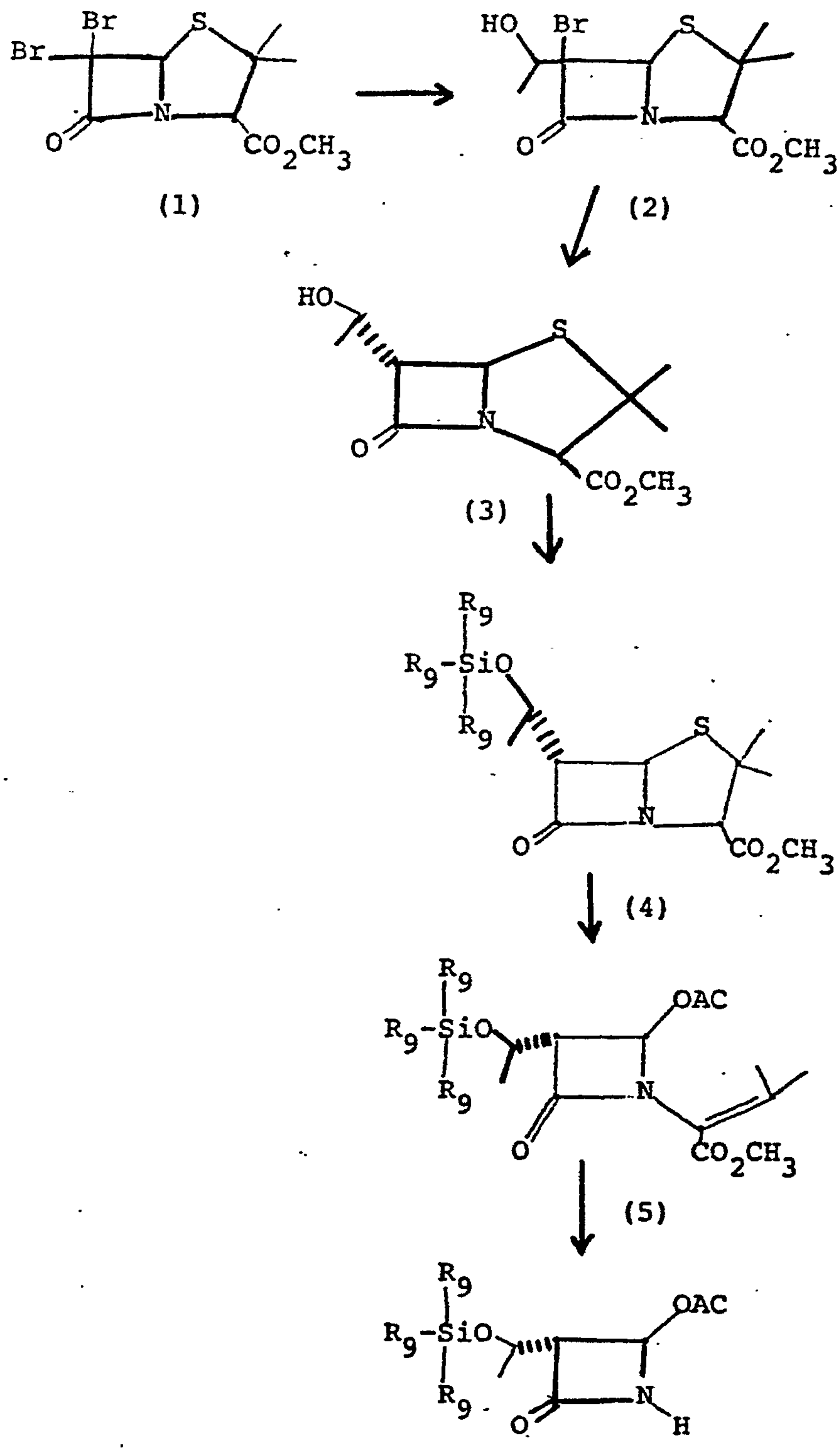

(1) (2) (3) (4) (5) (6)

Scheme B

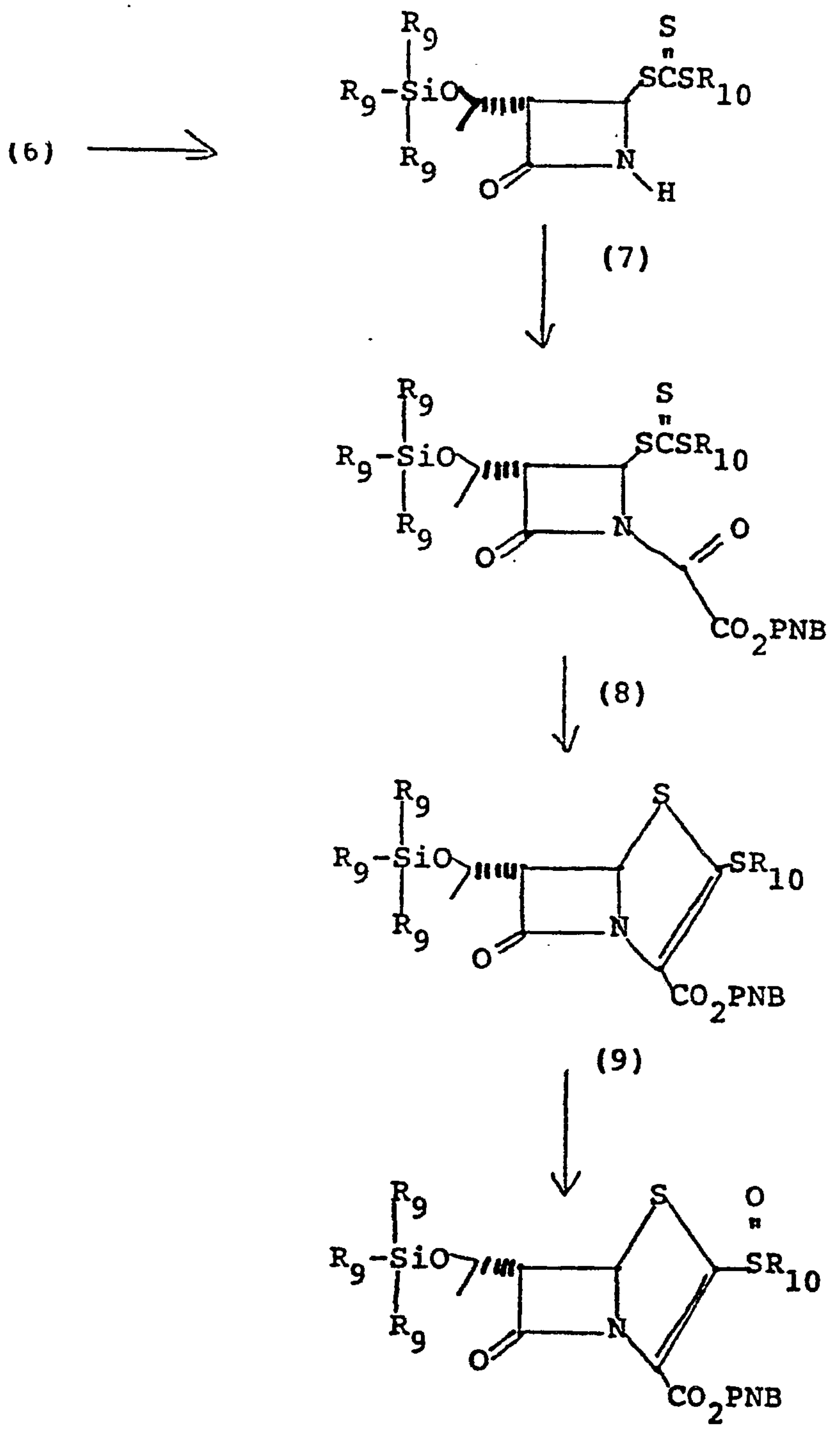

(6)

(7)

(8)

(9)

(10)

Scheme C

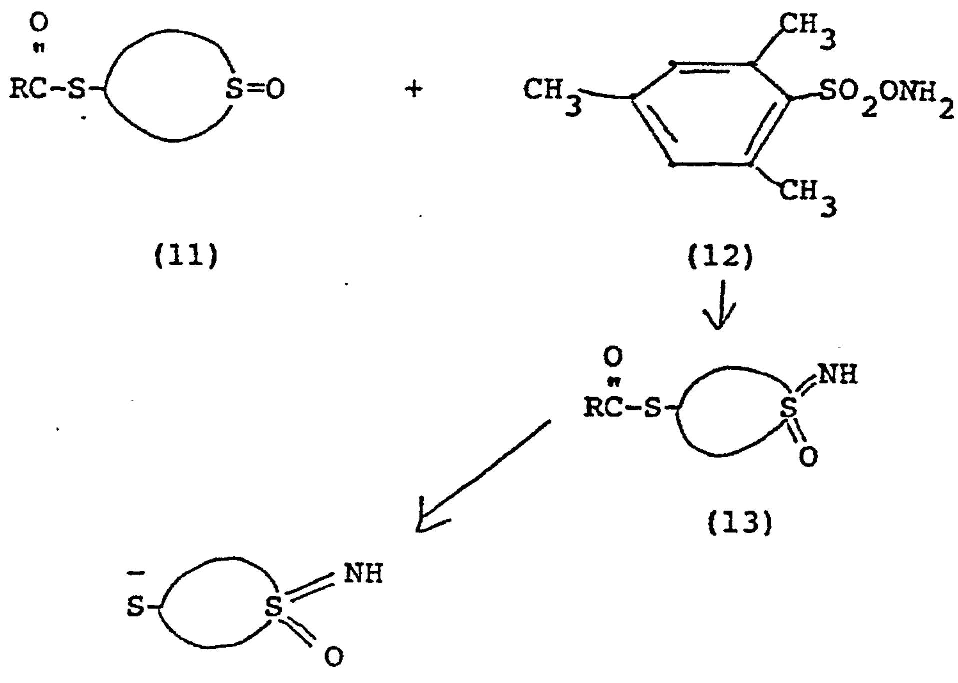

(11)

(12)

(13)

(13a)

Scheme D

(13a) + (10)

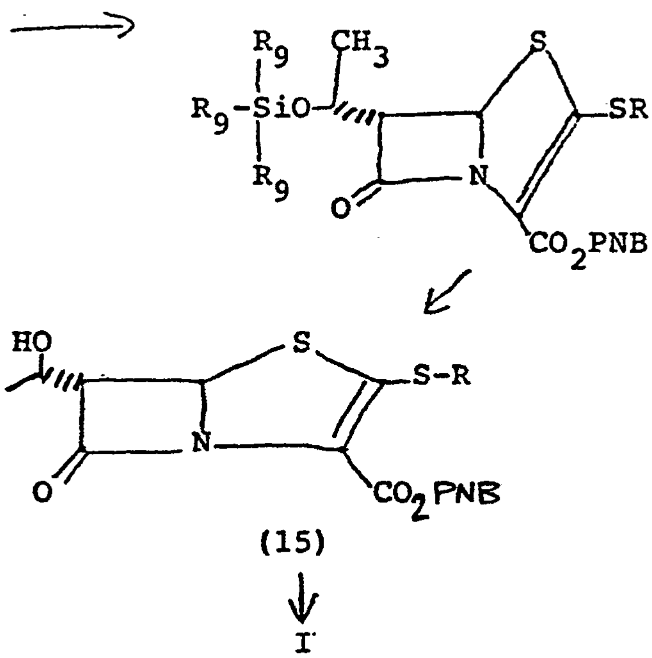

(14)

(15)

I

Scheme E

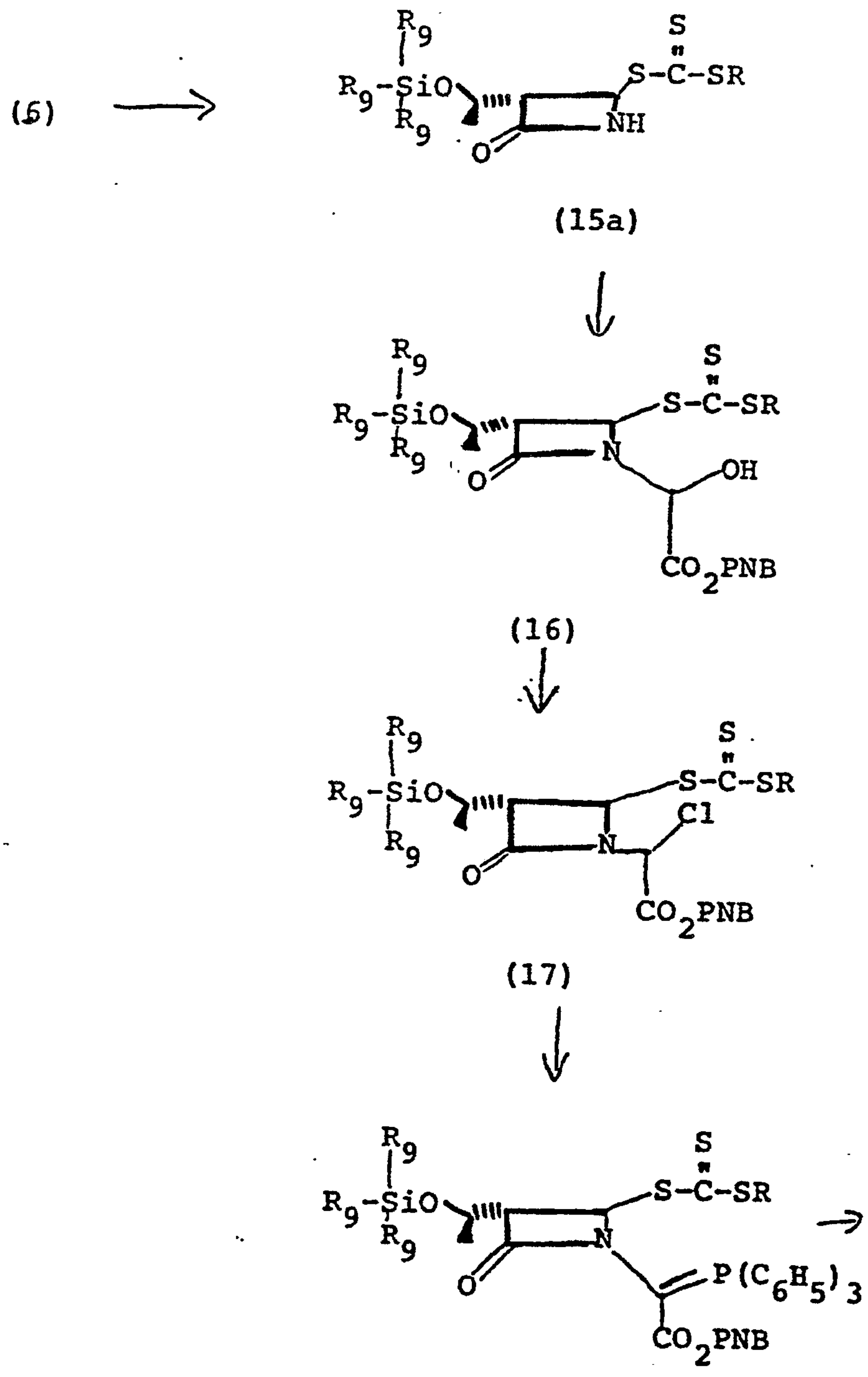

(6)

(15a)

(16)

(17)

(18)

I

As shown in Scheme A, the starting material for the synthesis of a compound of Formula I is the known dibromo penam of formula (1). The synthetic procedure is that of Yoshida et al., Chem. Pharm. Bull., 39, 2899-2909 (1981). The dibromo penam of formula (1) undergoes an exchange reaction with t-butyl magnesium chloride at a temperature of between about -90° C and -40° C, preferably at about -78° C, in a reaction-inert solvent, such as tetrahydrofuran, diethyl ether or toluene, preferably tetrahydrofuran. Other organometallic reagents may also be employed. The resultant reaction mixture is then treated with the appropriate aldehyde, e.g., acetaldehyde for the 1-hydroxyethyl derivative. The aldehyde is added at a

temperature between -80° C and -60° C, preferably at about -78° C if acetaldehyde is used.

The resultant bromo hydroxy penam (2) is then hydrogenated to remove the 6-bromo substituent. The hydrogenation reaction is carried out in the presence of a noble metal catalyst, such as palladium in a protic solvent such as 1:1 methanol-water or 1:1 tetrahydrofuran-water, preferably 1:1 methanol-water, at a pressure of about 1 to 4 atmospheres, preferably 4 atmospheres, and a temperature between 0° C and 30° C, preferably about 25° C.

The resulting alcohol of formula (3) can then be protected with a trialkylhalosilane of the formula:

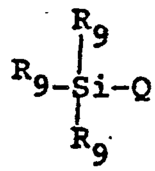

wherein $R_9$ at each occurrence is an alkyl of 1 to about 6 carbon atoms and Q is chloro, bromo or iodo. The protecting reaction is carried out in the presence of an amine proton acceptor, such as imidazole, in a polar, aprotic solvent, such as N,N-dimethylformamide, at a temperature range of from about 5° C to about 40° C, preferably at about 25° C. The trialkylsilyl protected compound is shown in formula (4).

Treatment of (4) with mercuric acetate in acetic acid at a temperature of about 90° C yields the olefin of formula (5).

In order to obtain the desired azetidinone of formula (6), the compound of formula (5) is ozonized in a reaction-inert solvent, such as dichloromethane, at a temperature of from about -80° C to about -40° C, preferably at about -78° C. The reaction product is then treated with an alkanol, such as methanol, to yield the azetidinone (6).

As shown in Scheme B, the compound of formula (6) is then treated with a trithiocarbonate salt of the formula $M^+R_{10}$-S-C(S)-$S^-$ wherein $R_{10}$ is alkyl of 1-4 carbon atoms, preferably ethyl, and M is a metal such as sodium or potassium, to obtain the compound of formula (7). The conversion of (6) to (7) is carried out in an organic solvent or water, preferably a mixture of water and dichloromethane, at a temperature range of 0° C to about 35° C, preferably at about 25° C.

The compound of formula (7) is then condensed with p-nitrobenzyl chloro-oxalate in the presence of a tertiary alkylamine wherein each alkyl group has, for example, 1-4 carbon atoms, such as, for example, ethyl - diisopropylamine, to obtain the compound of formula (8). This condensation reaction is carried out in a reaction-inert solvent, such as dichloromethane, at a temperature range of from about 5° C to about 25° C, preferably at about 10° C.

The resulting compound of formula (8) is then cyclized using a trialkyl phosphite wherein each alkyl group has 1 to about 4 carbon atoms, such as triethylphosphite, in a reaction-inert solvent, such as trichloromethane, at a temperature range of from about 40° C to about 80° C to yield the penem of formula (9).

The thiol compound of formula (9) is then oxidized to the corresponding sulfoxide compound of formula (10) with an oxidizing agent such as m-chloroperbenzoic acid, in a reaction-inert solvent, such as dichloromethane, at a temperature range of from about -10° C to about -30° C, preferably at about -20° C.

As is shown in Scheme D, the sulfoxide of formula (10) is then reacted with a thiacycloalkane of the formula $RS^-$ (13a). Thiacycloalkanes of the formula $RS^-$ are synthesized according to the procedure shown in Scheme C. In the first step, a 1-oxo-4-acylthio-1-thiacycloalkane of formula (11) is reacted with O-mesitylenesulfonylhydroxylamine (MSH) (12) in a reaction-inert solvent, such as dichloromethane, at room temperature to yield the 1-oxo-1-imino-4-acylthio-1-thiacycloalkane of formula (13) which is then hydrolyzed to $RS^-$ using sodium methoxide.

To obtain the compound of formula (15), the trialkylsilyl group is preferably removed prior to the hydrogenolysis to remove the PNB acid-protecting group. The trialkylsilyl group is removed with tetraalkylammonium fluoride in an ethereal solvent, such as tetrahydrofuran, at a temperature range of from about 15° C to about 40° C, preferably at about 25° C.

An alternate synthetic procedure is shown in Scheme E. The azetidinone of formula (6) is reacted with a trithiocarbonate of the formula $M^+$R-S-C(S)-$S^-$ wherein M is a metal such as sodium or potassium using the previously described procedure to prepare (7).

The resulting trithiocarbonate (15a) is treated with (p-nitrobenzyloxycarbonyl)(dihydroxy) methane in an aprotic solvent such as benzene, toluene or dimethylformamide, preferably benzene, at a temperature range of about 25-110° C, preferably about 80° C, to yield the alcohol of formula (16).

The corresponding chloride (17) is prepared from the alcohol (16) by treatment with thionyl chloride in a reaction-inert organic solvent, such as dichloromethane, in the presence of a hindered amine which serves as an acid acceptor, such as 2,6-lutidine at a temperature range of from about -10° to 75° C, preferably 0° C.

The chloride (17) is reacted with a triarylphosphine such as triphenylphosphine in a reaction inert solvent, such as tetrahydrofuran, in the presence of a tertiary amine, such as 2,6-lutidine, at a temperature of about 25° C to obtain the compound of formula (18) which is cyclized by refluxing in an aromatic solvent such as toluene to yield the penem of formula (11).

In those instances where $R_1$ is other than hydrogen, the desired compound can be prepared by methods well known to those skilled in the art. For instance, reacting the compound of formula I when $R_1$ is hydrogen with an sulfonylalkyl halide results in a compound wherein $R_1$ is a sulfonylalkyl group. When $R_1$ is hydrogen, reaction of the compound of formula I with an acyl chloride in the presence of a base, such as 4-dimethylaminopyridine results in a compound wherein $R_1$ is an alkylcarbonyl group. For preparation of those compounds wherein $R_1$ is an alkylaminocarbonyl group, the desired group is added by reaction with the appropriate isocyanate in the presence of a base. To prepare those compounds wherein $R_1$ is an alkoxycarbonyl group, the desired group is added by reaction with the appropriate chloroformate in the presence of a base.

Conversion of a compound of formula (15) to a compound of formula I, is accomplished using a conventional hydrogenolysis reaction, and it is carried out in conventional fashion for this type of transformation. Thus, a solution of a compound of the formula (15) is stirred or shaken under an atmosphere of hydrogen, or hydrogen mixed with an inert diluent such as nitrogen or argon, in the presence of a catalytic amount of a noble metal hydrogenolysis catalyst, such as a palladium-on-calcium carbonate or a palladium-on-celite (a diatomaceous earth) catalyst. Convenient solvents for this hydrogenolysis are lower alkanols, such as methanol ethers such as tetrahydrofuran and dioxan, low molecular weight esters, such as ethyl acetate and butyl acetate, water, and mixtures of these solvents. However, it is usual to choose conditions under which the starting material is soluble. The hydrogenolysis is usually carried out at room temperature and at a pressure from about 0.5 to about 5 kg/cm$^2$. The catalyst is usually present in an amount from about 10 percent by weight based on the starting material up to an amount equal in weight to the starting material, although larger amounts can be used. The reaction commonly takes about one hour after which the compound of formula I is recovered simply by filtration followed by removal of the solvent in vacuo. If palladium-on-calcium carbonate is used as the catalyst, the product is isolated as the calcium salt and if palladium-on-celite is employed, the product is isolated as the sodium salt.

The compounds of formula I can be purified by conventional methods for beta-lactam compounds. For example, the compound of formula I can be purified by gel filtration on Sephadex $^R$, or by recrystallization.

The compounds of formula I are acidic and will form salts with basic agents. Such salts are considered to be within the scope of this invention. These salts can be prepared by standard techniques, such as contacting the acidic and basic components, usually in a stoichiometric ratio, in an aqueous, non-aqueous or partially aqueous medium, as appropriate. They are then recovered by filtration, by precipitation with a non-solvent followed by filtration, by evaporation of the solvent, or, in the case of aqueous solutions, by lyophilization, as appropriate. Basic agents which are suitably employed in salt formation belong to both the organic and inorganic types, and they include ammonia, organic amines, alkali metal hydroxides, carbonates, bicarbonates, hydrides and alkoxides, as well as alkaline earth metal hydroxides, carbonates, hydrides and alkoxides. Representative examples of such bases are primary amines such as n-propylamine, n-butylamine, aniline, cyclohexylamine, benzylamine and octylamine; secondary amines such as diethylamine, morpholine, pyrrolidine and piperidine; tertiary amines such as triethylamine, N-ethylpiperidine, N-methylmorpholine, and 1,5-diazabicyclo[4,3,0]non-5-ene; hydroxides such as sodium hydroxide, potassium hydroxide, ammonium hydroxide, and barium hydroxide; alkoxides such as sodium ethoxide and potassium ethoxide; hydrides such as calcium hydride and sodium hydride; carbonates such as potassium carbonate and sodium carbonate; bicarbonates, such as sodium bicarbonate and potassium bicarbonate; and alkali metal salts of long-chain fatty acids, such as sodium 2-ethylhexanoate.

Preferred salts of the compounds of formula I are sodium, potassium and calcium salts.

The pharmaceutically-acceptable salts of formula I are those which are free of significant adverse side effects at the level of ordinary use and include, e.g., the sodium, potassium or calcium salts thereof.

The in vitro antibacterial activity of the compounds of formula I and salts thereof can be demonstrated by measuring their minimum inhibitory concentrations (MIC's) in mcg/ml against a variety of microorganisms. The procedure which is followed is the one recommended by the International Collaborative Study on Antibiotic Sensitivity Testing (Ericsson and Sherris, Acta, Pathologica et Microbiologia, Scandinav, Supp. 217, Section B: 64-68 [1971]), and employs brain heart infusion (BHI) agar and the inocula replicating

device. Overnight growth tubes are diluted 100-fold for use as the standard inoculum, (20,000 - 10,000 cells in approximately 0.002 ml are placed on the agar surface; 20 ml of BHI agar/dish). Twelve 2-fold dilutions of the test compound are employed, with initial concentration of the test drug being 200 mcg/ml. Single colonies are disregarded when reading plates after 18 hours at 37° C. The susceptibility (MIC) of the test organism is accepted as the lowest concentration of compound capable of producing complete inhibition of growth as judged by the naked eye.

The compounds of formula I, and the pharmaceutically-acceptable salts thereof, are suitable for the control of bacterial infections in mammals, including man. They will find use in the control of infections caused by susceptible bacteria in human subjects, e.g., infections caused by susceptible strains of Staphylococcus aureus.

The compounds of the present invention can be administered orally or parenterally, i.e., intramuscularly, subcutaneously, intraperitoneally or intravenously, alone or combined with a pharmaceutically-acceptable carrier according to standard pharmaceutical practice. The ratio of active ingredient to carrier will depend upon the chemical nature, solubility and stability of the active ingredient, as well as the dosage contemplated. The ratio of the pharmaceutically-acceptable carrier to the penem compound will normally be in the range of from 1:10 to 4:1. For oral administration, the compounds of this invention can be used in the form of tablets, capsules, lozenges, troches, powders, syrups, elixirs, aqueous solutions and suspensions, and the like. In the case of tablets, carriers which can be used include lactose, sodium citrate and salts of phosphoric acid. Various disintegrants such as starch and lubricating agents, such as magnesium stearate, sodium lauryl sulfate and talc, are commonly used in tablets. Useful diluents for capsules include lactose and high molecular weight polyethylene glycols. When aqueous suspensions are required for oral use, the active ingredient is combined with emulsifying and suspending agents. Sweetening and/or flavoring agents can be added. For parenteral administration, sterile solutions of the active ingredient are usually prepared, and the pH of the solutions is suitably adjusted and buffered. For intravenous use, the total concentration of solutes should be controlled to render the preparation isotonic. The prescribing physician will ultimately determine the appropriate dose for a given human subject, and this can be expected to vary according to the age, weight, and response of the individual patient, as well as the nature and severity of the patient's symptoms. The compounds of formula I will normally be used orally at dosages in the range of from about 10 to about 200 mg. per kilogram of body weight per day. In some cases, it may be necessary to use dosages outside these limits.

The following Examples serve to illustrate the invention and are not to be construed as limiting the invention. Infra-red (IR) spectra were measured either as potassium bromide discs, (KBr disc), Nujol mulls or as solutions in chloroform, ($CHCl_3$), methylene, chloride ($CH_2Cl_2$) or dimethyl sulfoxide (DMSO) and diagnostic absorption bands are reported in either microns or wave numbers ($cm^{-1}$). Nuclear magnetic resonance (NMR) spectra were measured for solutions in deuterochloroform ($CDCl_3$), perdeutero methanol ($CD_3OD$) or perdeuterodimethyl sulfoxide (DMSO-$d_6$), or mixtures thereof, and peak positions are expressed in parts per million downfield from tetramethylsilane. The following abbreviations for peak shapes are used: s, singlet, d, doublet, t, triplet, q, quartet, m, multiplet, b, broad, c, complex. The abbreviations "ss" and "sss" denote that a particular proton appeared as two' or three singlets respectively owing to the presence of diastereoisomers. Throughout the Examples, the abbreviation "PNB" represents the p-nitrobenzyl group.

EXAMPLE 1

**Preparation of cis-1-oxo-1-imino-4-acetylthio-1-thiacyclohexane**

Under a nitrogen atmosphere were combined 1.0 g of cis-1-oxo-4-acetylthio-1-thiacyclohexane and 10 ml of methylene chloride. To the resulting solution was added over a 5 minute period a 15 ml $CH_2Cl_2$ solution of 1.34 g of O-mesitylenesulfonylhydroxylamine (MSH). A precipitate slowly formed. After 2 hours, an additional 0.9 g of MSH in 10 ml $CH_2Cl_2$ was added. After stirring at room temperature overnight, the reaction mixture was diluted to 80 milliliters with $CH_2Cl_2$, 2.0 g solid sodium bicarbonate was added and then 15 ml of water. The final pH was 8.0. The $CH_2Cl_2$ layer was separated, dried over sodium sulfate and concentrated to yield 1.0 g of a viscous amber colored oil which was further purified via silica gel column chromatography using 95/5 ethyl acetate/methanol as eluant to yield 0.43 g (37% yield) of product as a white solid.

250 MHZ PNMR $CDCl_3$

1.95 to 2.75 (complex multiplet 5H), 2.35 (s,3H), 2.95-3.25 (m,4H); 3.67-3.8 (m,1H).

EXAMPLE 2

**Preparation of trans-1-oxo-1-imino-4-acetylthio-1-thiacyclopentane**

Similar to Example 1 except trans-1-oxo-4-acetylthio-1-thiacyclopentane was used as the starting material. The final yield was 51% of a light oil.
250 MHZ PNMR $CDCl_3$
2.1-2.4 (complex m,1H); 2.35 (s,3H); 2.55-2.72 (m,1H); 2.7-2.95 (broad,1H); 3.0-3.1 (m,1H); 3.1-3.4 (complex m,2H); 3.6-3.7 (m,1H); 4.15-4.33 (complex m,1H).

EXAMPLE 3

**Preparation of trans-1-oxo-1-imino-4-acetylthio-1-thiacyclohexane**

Similar to Example 2 except trans-1-oxo-4-acetylthio-1-thiacyclohexane was used as the starting material. The final yield was 26.7% of a white solid.
250 MHZ PNMR $CDCl_3$
2.39 (s) and complex multiplet (2.16-2.55, 8H); 3.1-3.26 (m,4H); 3.72-3.85 (m,1H).

EXAMPLE 4

**Preparation of cis-1-oxo-1-imino-4-acetylthio-1-thiacyclopentane**

Similar to Example 3 except cis-1-oxo-4-acetylthio-1-thiacyclopentane was used as the starting material. The final yield was 49% of a viscous oil.
T-60 NMR $CDCl_3$
2.4 (s,3H). The rest of the spectrum was a series of complex multiplets from 2.0 ppm to 4.7 ppm.
High resolution Mass Spec 193.0231 $C_6H_{11}NO_2S_2$

EXAMPLE 5

**Preparation of PNB(5R,6S)-2-(trans-1-oxo-1-imino-1-thiacyclohex-4-ylthio)-6-[(R)-1-t-butyldimethyl silyloxyethyl]-2-penem-3-carboxylate**

Under a nitrogen atmosphere, 0.247 g (0.0012 mole) of trans-1-oxo-1-imino-4-acetylthio-1-thiacyclohexane was dissolved in 15 ml of absolute ethanol and this solution was cooled to -15°C. 1.2 ml of 1 molar sodium ethoxide in ethanol was then added and the mixture was cooled down to -20°C. Hydrolysis was monitored by TLC and was complete in 1.5 to 2 hours. The solution was cooled down to -60°C and transferred via canula and nitrogen pressure to another reaction vessel containing a 30 ml tetrahydrofuran solution of 0.648 g (0.0012 mole) of p-nitrobenzyl ( 5R,6S)-6-[(R)-1-t-butyldimethylsilyloxyethyl-2-ethylsulfinyl-2-penem-3-carboxylate cooled to -60C. The reaction was stirred at -60°C for 45 min. then 0.25 ml of acetic acid in 5 ml tetrahydrofuran was added. The reaction was warmed to room temperature and the tetrahydrofuran and ethanol were removed under reduced pressure. The residue was then taken up in 150 ml of ethyl acetate and washed three times with 25 ml of $H_2O$, once with 25 ml of a saturated sodium bicarbonate solution, once with 25 ml $H_2O$, and once with 25 ml brine. The ethyl acetate solution was dried over sodium sulfate, filtered, concentrated and chromatographed on 125 g of silica gel using 95/5 ethyl acetate/methanol as eluent to yield 0.46 g of pale yellow solids (61% yield).
IR (KBr) 1791 $cm^{-1}$
250 PNMR $CDCl_3$
0.03 (s,3H); 0.06 (s,3H); 0.82 (s,9H); 1.25 (d,3H); 2.2-2.65 (complex m,5H); 2.96-3.15 (m,2H); 3.175-3.35 (m,2H); 3.43-3.55 (m,1H); 3.75 (dd,1H); 4.2-4.33 (m,1H); 5.2 (d,1H); 5.41 (d,1H); 5.68 (d,1H); 7.62 (d,2H); 8.21 (d,2H).

EXAMPLE 6

**Preparation of PNB(5R,6S)-2-(cis-1-oxo-1-imino-1-thiacyclohex-4-ylthio)-6[(R)-1-t-butyldimethylsilyloxyethyl]-2-penem-3-carboxylate**

Similar to Example 5 except the starting material was the cis isomer. The final yield was 64% of a light yellow powder.

250 MHZ PNMR DMSO-$d_6$
0.01 (s,3H); 0.033 (s,3H); 0.77 (s,9H); 1.2 (d,3H); 1.95-2.6 (complex m,5H); 2.90-3.25 (complex m,4H); 3.52-3.67 (m,1H); 4.03 (d,1H); 4.17 4.3 (m,1H); 5.26 (d,1H); 5.4 (d,1H); 5.77 (d,1H); 7.7 (d,2H); 8.23 (d,2H).

EXAMPLE 7

**Preparation of a diastereomeric mixture of PNB(5R,6S)-2-(trans-1-oxo-1-imino-1-thiacyclopent-3-ylthio)-6-[(R)-1-t-butyldimethylsilyloxyethyl-2-penem-3-carboxylate**

Similar to Example 5 except the starting material was the trans-cyclopentylthio compound. The final yield was 54% of the diastereomers.
250 MHZ PNMR $CDCl_3$
0.03 (s,3H); 0.07 (s,3H); 0.82 (s,9H); 1.25 (d,3H); 2.17-2.42 (complex m,1H); 2.63-2.85 (complex m,lH); 2.96 (broad s,1H); 3.05-3.29 (complex m,2H); 3.33-3.48 (m,1H); 3.6-3.84 (m,1H); 3.785 (2dd,1H); 3.96.-4.14 (m,1H); 4.22-4.34 (m,1H); 5.225 (d,1H); 5.42 (d,1H); 5.71 & 5.72 (2d,1H); 7.616 (d,2H); 8.21 (d,2H).

EXAMPLE 8

**Preparation of a diastereomeric mixture of PNB(5R,6S) 2-(cis-1-oxo-1-imino-1-thiacyclopent-3-ylthio-6-[(R)-1-t-butyldimethylsilyloxyethyl-2-penem-3-carboxylate**

Similar to Example 5 except the starting material was the cis-cyclopentylthio compound. The final yield was 50% of the diastereomeric mixture. The following IR and NMR data are for the less polar isomer.
IR (KBr) 1791
250 PNMR $CDCl_3$
0.04 (s,3H); 0.085 (s,3H); 0.835 (s,9H); 1.26 (d,3H); 2.23-2.45 (complex m,2H); 3.1-3.5 (complex m,3H); 3.62-3.8 (m,1H); 3.78 (dd,1H); 3.92-4.1 (m,1H); 4.22-4.34 (m,1H); 5.22 (d,1H); 5.43 (d,1H); 5.71 (d,1H); 7.62 (d,2H); 8.23 (d,2H).

EXAMPLE 9

**Preparation of a diastereomeric mixture of PNB( 5R,6S)-2-(trans-1-oxo-1-acetylimino-1-thiacyclopent-3-ylthio)-6[(R)-1-t-butyldimethylsilyloxyethyl-2-penem-3-carboxylate**

The following is an example of the acylation of a penem of the present invention.

Under a nitrogen atmosphere were combined 0.15 g (0.00245 mole) of the compound of Example 7 and 10 ml of methylene chloride. The solution was cooled to 0°C and 0.060 g (0.00049 mole) of 4-dimethylaminopyridine was added. The solution was stirred at 0°C and a 1 ml solution of 0.0175 ml (0.000245 mole) of acetyl chloride in methylene chloride was added dropwise over a 2 to 3 minute period. After the addition was complete, the solution was stirred for an additional 10 minutes. Thin layer chromatography (TLC) showed the reaction was complete. The reaction mixture was diluted to 100 ml with methylene chloride, washed with dilute acid (0.01 M HCl), $H_2O$, and brine, dried over sodium sulfate, filtered and concentrated to yield the product as a gum. The isomers were separated by chromatography on silica gel using 60/40 ethyl acetate/hexane as eluent to yield 68 mg of the less polar isomer and 63 mg of the more polar isomer in an overall yield of 82%.
250 MHZ PNMR $CDCl_3$ (less polar isomer)
0.025 (s,3H); 0.06 (s,3H); 0.82 (s,9H); 1.26 (d,3H); 2.125 (s,3H); 2.2-2.43 (m,1H; 2.73-2.89 (m,1H); 3.2-3.35 (m,1H); 3.5-3.6 (m,1H); 3.72-3.9 (m,2H); 3.8 (dd,1H); 4.03-4.2 (m,1H); 4.22-4.35 (m,1H); 5.22 (d,1H); 5.41 (d,1H); 5.71 (d,1H); 7.62 (d,2H); 8.41 (d,1H).

EXAMPLES 9a-c

Using the procedure of Example 9, the following compounds were obtained:

| Example # | Compound | Yield (%) |
|---|---|---|
| 9a | PNB(5R,6S)-2-(cis-1-oxo-1-acetylimino-1-thiacyclopent-3-ylthio)-6-[(R)-1-t-butyldimethylsilyloxyethyl]-2-penem-3-carboxylate (diastereomeric mixture) | 98 |
| 9b | PNB(5R,6S)-2-(cis-1-oxo-1-acetylimino-1-thiacyclohex-4-ylthio)-6-[(R)-1-t-butyldimethylsilyloxyethyl]-2-penem-3-carboxylate | 84 |
| 9c | PNB(5R,6S)-2-(trans-1-oxo-1-acetylimino-1-thiacyclohex-4-ylthio)-6-[(R)-1-t-butyldimethylsilyloxyethyl]-2-penem-3-carboxylate | 87 |

250 NMR $CDCl_3$
0.06 (s,3H); 0.09 (s,3H); 0.84 (s,9H); 1.29 (d,3H); 2.16 (s,3H); 2.23-2.46 (m,2H); 2.52-2.74 (m,2H); 3.24-3.42 (m,2H); 3.51-3.62 (m,1H); 3.7-3.9 (complex m,3H); 4.26-4.36 (m,1H); 5.24 (d,1H); 5.47 (d,1H); 5.74 (d,1H); 7.67 (d,2H); 8.26 (d,2H).

EXAMPLE 10

**Preparation of PNB(5R,6S)-2-(trans-1-oxo-1-methylsulfonylimino-1-thiacyclopent-3-yl)-6-[(R)-1-t-butyldimethylsilyloxyethyl]-2-penem-3-carboxylate**

Similar to Example 9, using methanesulfonyl chloride, except triethylamine was used as the base instead of dimethylaminopyridine. The final yield, after chromatography on silica gel using 7:4 ethyl acetate/hexane as eluent, was 72%.

250 PNMR $CDCl_3$ (less polar (TLC) isomer)
0.04 (s,3H); 0.083 (s,3H); 0.84 (s,9H); 1.26 (d,3H); 2.25-2.47 (m,1H); 2.75-2.94 (m,1H); 3.15 (s,3H); 3.25-3.46 (m,1H); 3.62-3.73 (m,1H); 3.815 (dd,1H); 3.9-4.18 (complex m,2H); 4.22-4.35 (m,1H); 5.24 (d,1H); 5.43 (d,1H); 5.725 (d,1H); 7.625 (d,2H); 8.225 (d,2H).

250 PNMR $CDCl_3$ (more polar (TLC) isomer)
0.04 (s,3H); 0.075 (s,3H); 0.825 (s,9H); 1.25 (d,3H); 2.22-2.4 (m,1H); 2.7-2.85 (m,1H); 3.133 (s,3H) 3.3-3.466 (m,1H); 3.66-3.775 (m,1H); 3.8 (dd,1H); 3.89-4.02 (m,1H); 4.02-4.2 (complex m,2H); 4.2-4.35 (m,1H); 5.24 (d,1H); 5.425 (d,1H); 5.75 (d,1H); 7.616 (d,2H); 8.216 (d,2H).

EXAMPLES 10a-b

Following the procedures of Example 10, the following compounds were prepared:

| Example # | Compound | Yield (%) |
|---|---|---|
| 10a | PNB(5R,6S)-2-(cis-1-oxo-1-methylsulfonylimino-1-thiacyclopent-3-ylthio)-6-[(R)--1-t-butyldimethyl-silyloxyethyl]-2-penem-3-carboxylate (diastereomeric mixture) | 84 |
| 10b | PNB(5R,6S)-2-(cis-1-oxo-1-methylsulfonylimino-1-thiacyclohex-4-ylthio)-6-[(R)--1-t-butyldimethylsilyloxyethyl]-2-penem-3-carboxylate | 89 |

EXAMPLE 11

**Preparation of PNB(5R,6S)-2-(trans-1-oxo-1-methoxycarbonylimino-1-thiacyclopent-3-ylthio)-6-[(R)-1-t-butyldimethylsilyloxyethyl]-2-penem-3-carboxylate**

Similar to Example 9, except N,N diisopropylethylamine was used as the base and both an excess of amine and methyl chloroformate were used to get the reaction to go to completion. The isomers were separated by column chromatography using 60/40 ethyl acetate/hexane as the eluent and the final yield was 96%.

250 PNMR $CDCl_3$ (more polar (TLC) isomer)

0.03 (s,3H); 0.07 (s,3H); 0.825 (d,9H); 1.25 (d,3H); 2.15-2.35 (m,1H); 2.675-2.85 (m,1H); 3.225-3.375 (m,1H); 3.5-3.96 (complex m's,3H); 3.725 (s,3H); 4.0-4.2 (complex m,3H); 4.2-4.33 (m,1H); 5.225 (d,1H); 5.4 (d,1H); 5.733 (d,1H); 7.6 (d,2H); 8.22 (d,2H).

EXAMPLE 12

**Preparation of PNB(5R,6S)-2-(trans-1-oxo-1-N-methylcarbamoylimino-1-thiacyclopent-3-ylthio)-6-1-(R)-1-t-butyldimethylsilyloxyethyl]-2-penem-3-carboxylate**

Under a nitrogen atmosphere were confined 0.15 g (0.000245 mole) of the compound of Example 7, 10 ml of methylene chloride, 0.0432 ml (0.000735 mole) of methylisocyanate, and 20 mg of 4-dimethylaminopyridine. The mixture was heated to reflux and after four hours, the reaction was complete as determined by TLC. The reaction mixture was diluted to 100 ml with methylene chloride, washed with 20 ml of 0.05 N HCl, 25 ml $H_2O$, and 25 ml brine, dried over sodium sulfate, filtered, concentrated. The product was purified by column chromatography using 75 g of silica gel and eluting with 9:1 ethyl acetate/methylene chloride to yield 0.108 g of light brown foam (66% yield).

250 PNMR $CDCl_3$

0.03 (s,3H); 0..066 (s,3H); 0.825 (s,9H); 1.25 (d,3H); 2.15-2.45 (m,1H); 2.77 and 2.79 (2s,3H); 2.6-2.95 (m,1H); 3.17-3.35 (m,1H); 3.57-3.95 (complex m,3H); 3.98-4.2 (complex m,2H); 4.2-4.35 (m,1H); 5.0 (broad singlet,1H); 5.22 (d,1H); 5.43 (d,1H); 5.712 and 5.725 (2d,1H); 7.61 (d,2H); 8.21 (d,2H).

EXAMPLE 13

**Preparation of PNB(5R,6S)-2-(trans-1-oxo-1-imino-1-thiacyclohex-4-ylthio)-6-[(R)-1-hydroxyethyl]-2-penem-3-carboxylate**

The following is a typical procedure for the desilylation of the compounds of the present invention.

Under a nitrogen atmosphere were combined 0.12 g (0.00019 mole) of the compound of Example 5, 2 ml anhydrous tetrahydrofuran, 0.12 ml (0.002 mole) acetic acid, and 0.8 ml of 1M tetrabutylammonium fluoride in THF. The mixture was allowed to stir at room temperature overnight. The product which had precipitated out of solution was filtered, washed three times with 1 ml THF and dried to yield 50 mg of a pale yellow solid (51.5% yield).

IR (KBr) 1770

250 PNMR DMSO-$d_6$

1.2 (d,3H); 1.92-2.16 (m,2H); 2.2-2.55 (m,2H); 2.95-3.35 (complex m's,4H); 3.5-3.68 (m's,2H); 3.925 (dd,1H); 3.95-4.075 (m,1H); 5.25 (d,1H); 5.315 (d,1H); 5.46 (d,1H); 5.79 (d,1H); 7.7 (d,2H); 8.26 (d,2H).

In those cases where the product does not precipitate out, the workup was as follows: The reaction was quenched by pouring into 100 ml ethyl acetate, the organic layer was washed 3 times with 10 ml $H_2O$, once with 20 ml of potassium bicarbonate, once with 10 ml of $H_2O$, and once with 25 ml brine. The ethyl acetate solution was then dried over sodium sulfate, filtered and concentrated to yield a product which could be further purified by column chromatography.

EXAMPLE 14

Using the procedure of Example 13, the following desilylated compounds were prepared.

| Desilylated Compound of | 250 PNMR | Yield(%) |
|---|---|---|
| Example 6[1] PNB(5R,6S)-2-(cis-1-oxo-1-imino-1-thiacyclohex-4-ylthio)-6-[(R)-1-hydroxyethyl]-2-penem-3-carboxylate | 1.17(d,3H); 1.95-2.65 (complex m's,4H); 2.9-3.45 (complex m's,4H); 3.52-3.68 (m,1H); 3.8 (broad s,1H); 3.9 (dd,1H); 3.95-4.09 (m,1H); 5.25 (broad s,1H); 5.3 (d,1H); 5.45 (d,1H); 5.78 (d,1H); 7.7 (d,1H); 8.25 (d,2H). | 82 |
| Example 8[1] (from more polar silyl ester) PNB(5R,6S)-2-(cis-1-oxo-1-imino-1-thiacyclopent-3-ylthio)-6-[(R)-1- | 1.37 (d,3H); 2.15-2.45 (complex m,1H); 2.52 (broad s,2H); 2.56-2.85 (complex m,1H); | 25 |

| | | |
|---|---|---|
| hydroxyethyl]-2-penem-3-carboxylate | 3.1-3.48 (complex m's,3H); 3.68-3.84 (m's,2H); 3.95-4.08 (m,1H); 4.2-4.35 (m,1H); 5.22 (d,1H); 5.46 (d,1H); 5.75 (d,1H); 7.62 (d,2H); 8.22 (d,2H). | |
| Example 8[1] (from less polar silyl ester) PNB(5R,6S)-2-(cis-1-oxo-1-imino-1-thiacyclopent-3-ylthio)-6-[(R)-1-hydroxyethyl]-2-penem-3-carboxylate | | 22 |
| Example 7[1] (diastereo-mixture) PNB(5R,6S)-2-(trans-1-oxo-1-imino-1-thiacyclopent-3-ylthio)-6-[(R)-1-hydroxyethyl]-2-penem-3-carboxylate | 1.18 (d,3H); 2.04-2.26 (complex m,1H); 2.5-2.8 (complex m,1H); 3.05-3.33 (complex m's,2H); 3.45-4.28 (series of m's,6H); 5.25 (d,1H); 5.31 (d,1H); 5.46 (d,1H); 5.805 (broad s,1H); 7.7 (d,2H); 8.26 (d,2H). | 36 |
| Example 9a[2] (diastereo-mixture) | 1.38 (d,3H); 1.9 (broad s,1H); | 66 |

| | | |
|---|---|---|
| PNB(5R,6S)-2-(cis-1-(1-oxo-1-acetylimino-1-thiacyclopent-3-ylthio)-6-[(R)-1-hydroxyethyl]-2-penem-3-carboxylate) | 2.14 (s,3H); 2.25-2.5 (complex m,1H); 2.65-2.87 (complex m,1H); 3.16-3.315 (m,1H); 3.36-3.5 (m,1H); 3.56-3.72 (m,1H); 3.79-3.86 (m,1H); 4.05-4.44 (2 complex m's,3H); 5.24 (d,1H); 5.48 (d,1H); 5.74 and 5.76 (2d,1H); 7.63 (d,2H); 8.23 (d,2H). | |
| Example 9[2] (from less polar silyl ester) PNB(5R,6S)-2-(trans-1-oxo-1-acetylimino-1-thiacyclopent-3-ylthio)-6-[(R)-1-hydroxyethyl]-2-penem-3-carboxylate | 1.4 (d,3H); 1.94 (d,1H); 2.125 (s,3H); 2.225-2.4 (m,1H); 2.73-2.9 (m,1H); 3.22-3.35 (m,1H); 3.525-3.625 (m,1H); 3.84 (dd,1H); 3.7-3.925 (m,2H); 4.05-4.20 (m,1H); 4.20-4.35 (m,1H); 5.24 (d,1H); 5.475 (d,1H); 5.75 (d,1H); 7.625 (d,2H); 8.24 (d,2H). | 89 |
| Example 9[2] (from more polar silyl ester) | 1.37 (d,3H); 1.98 (d,1H); 2.12 (s,3H); | 80 |

| | | |
|---|---|---|
| | 2.15-2.34 (m,1H); 2.675-2.85 (m,1H); 3.2-3.36 (m,1H); 3.54-3.64 (m,1H); 3.7-3.85 (m,1H); 3.805 (dd,1H); 3.95-4.2 (complex m,2H); 4.2-4.35 (m,1H); 5.225 (d,1H); 5.475 (d,1H); 5.765 (d,1H); 7.62 (d,2H); 8.225 (d,2H). | |
| Example 11[2] (from less polar silyl ester) PNB(5R,6S)-2-(trans-1-oxo-1-methoxycarbonylimino-1-thiacyclopent-3-ylthio)-6-[(R)-1-hydroxyethyl)-2-penem-3-carboxylate) | 1.38 (d,3H); 1.9-2.08 (broad s,1H); 2.2-2.43 (complex m,1H); 2.72-2.94 (complex m,1H); 3.24-3.40 (complex m,1H); 3.55-3.685 (m,1H); 3.74 (s,3H); 3.75-4.0 (complex m's,3H); 4.05-4.23 (m,1H); 4.23-4.38 (m,1H); 5.24 (d,1H); 5.46 (d,1H); 5.74 (d,1H); 7.62 (d,2H); 8.22 (d,2H). | 75.8 |
| Example 11[2] (from more polar silyl ester) | 1.40 (d,3H); 1.9 (d,1H); | 71 |

| | | |
|---|---|---|
| | 2.15-2.40 (complex m,1H);<br>2.7-2.9 (complex m,1H);<br>3.25-3.4 (m,1H);<br>3.55-3.72 (m,1H);<br>3.74 (s,3H);<br>3.7-3.95 (m's,2H);<br>4.0-4.22 (m,2H);<br>4.22-4.375 (m,1H);<br>5.24 (d,1H);<br>5.475 (d,1H);<br>5.76 (d,1H);<br>7.62 (d,2H);<br>8.225 (d,2H). | |
| Example 12[2] (mixture) PNB(5R,6S)-2-(trans-1-oxo-1-N-methylcarbamoylimino-1-thiacyclopent-3-ylthio)-6-[(R)-1-hydroxyethyl]-2-penem-3-carboxylate | 1.38 (d,3H);<br>1.6 (broad s,1H);<br>2.1-2.4 (complex m,1H);<br>2.75-2.765 (2s,3H);<br>2.6-2.95 (complex m,1H);<br>3.16-3.35 (m,1H);<br>3.55-4.35 (series complex m's,7H);<br>5.05 (d,1H);<br>5.24 (d,1H);<br>5.46 (d,1H);<br>5.74-5.766 (2d,1H);<br>7.62 (d,2H);<br>8.22 (d,2H). | 84 |
| Example 10[1] (from more polar silyl ester) PNB(5R,6S)-2-(trans- | $CH_3$ 1.18<br>(O)S(O)-CH 3.05<br>H-5 5.84 | 40 |

| | | |
|---|---|---|
| 1-oxo-1-methyl-sulfonylimino-1-thia-cyclopent-3ylthio)-6-[(R)-1-hydroxy-ethyl-2-penem-3-carboxylate) | 7.7 (d,2H); 8.24 (d,2H). | |
| Example 10[1] (from less polar silyl ester) PNB(5R,6S)-2-(trans-1-oxo-1-methylsulfonyl imino-1-thiacyclopent-3-ylthio)-6-[(R)-1-hydroxyethyl]-2-penem-3-carboxylate | | 14 |

1 DMSO - $D_6$

2 $CDCl_3$

**EXAMPLE 15**

**Preparation of sodium (5R,6S)-2-(trans-1-oxo-1-imino-1-thiacyclohex-4-ylthio)-6-[(R)-1-hydroxyethyl]-2-penem-3-carboxylate**

The following is a typical procedure for the removal of the p-nitrobenzyl group.

In a 250 ml Parr bottle were combined 10 ml THF and 40 mg (0.000078 mole) of the penem of Example 13. One ml of $H_2O$ was added to the suspension and the bottle was swirled to achieve a clear solution. To this solution was added a suspension of 40 mg of 10% Pd- on-celite in 10 ml $H_2O$, the pH of which had been adjusted to 7.0 with dilute HCl. The resulting mixture was hydrogenated at 60 PSI for 30 minutes at which time the pH was checked and adjusted from 4.8 to 6.8 using an aqueous sodium bicarbonate solution. 40 mg of fresh catalyst was added and the hydrogenation was continued at 60 PSI for 1 to 1¼ hours. The reaction was checked for completion by TLC.

The reaction mixture was worked up as follows: The pH was adjusted to 6.5 using an aqueous sodium bicarbonate solution. The catalyst was removed by filtration through a millipore filtration apparatus. The catalyst pad was washed with a 1:1 THF/$H_2O$ mixture and washes were combined with the original filtrate. The THF was removed on a rotary evaporator under reduced pressure and the resulting aqueous solution was extracted twice with 20 ml portions of ethyl acetate. The solvents were removed on a rotary evaporator. The pH of the resulting solution was 6.5. The aqueous portion was then lyophilized to yield 21 mg of a white solid (67.7%) yield.

IR (KBr) 1774

250 PNMR $D_2O$

1.26 (d,3H); 2.06-2.275 (m,2H); 2.35-2.26 (m,2H); 3.15-3.48 (m,4H); 3.5-3.65 (m,1H); 3.9 (dd,1H); 4.175-4.3 (m,1H); 5.67 (d,1H).

EXAMPLE 16

Using the procedures of Example 15, there were prepared the sodium salts of the following compounds of Formula I:

| Compound | NMR ($D_2O$) | Yield(%) |
|---|---|---|
| Sodium(5R,6S)-2-(trans-1-oxo-1-acetylimino-1-thiacyclohex-4-ylthio)-6-[(R)-1-hydroxyethyl]-2-penem-3-carboxylate | 1.28 (d,3H); 2.1125 (s,3H); 2.05-2.3 (m,2H); 2.425-2.675 (m,2H); 3.43-3.675 (m,2H); 3.75-3.95 (m,2H); 3.915 (dd,1H); 4.15-4.275 (m,1H); 5.67 (d,1H). | 58 |
| Sodium(5R,6S)-2- | 1.3 (d,3H); | 67.5 |

| | | |
|---|---|---|
| (cis-1-oxo-1-acetylimino-1-thiacyclohex-4-ylthio-6-[(R)-1-hydroxyethyl]-2-penem-3-carboxylate | 2.11 (s,3H); 2.2-2.44 (complex m,2H); 2.44-2.66 (complex m,2H); 3.55-3.85 (complex m,5H); 3.95 (dd,1H); 4.18-5.32 (m,1H); 5.685 (d,1H). | |
| Sodium(5R,6S)-2-(trans-1-oxo-1-acetylimino-1-thiacyclopent-3-ylthio)-6-[(R)-1-hydroxyethyl]-2-penem-3-carboxylate (from less polar silyl ester) | 1.295 (d,3H); 2.12 (s,3H); 2.23-2.42 (m,1H); 2.78-2.96 (m,1H); 3.47-3.63 (m,1H); 3.95 (dd,1H) superimposed on complex m; 3.73-4.1 (3H); 4.18-4.4 (complex m's,2H); 5.71 (d,1H). | 77 |
| Sodium(5R,6S)-2-(trans-1-oxo-1-acetylimino-1-thiacyclopent-3-ylthio)-6-[(R)-1-hydroxyethyl]-2-penem-3-carboxylate (from more polar silyl ester) | 1.3 (d,3H); 2.11 (s,3H); 2.15-2.4 (complex m,1H); 2.73-2.95 (complex m,1H); 3.48-3.65 (m,1H); 3.78-4.0 (complex m,2H); 3.94 (dd,1H); 4.02-4.17 (m,1H); 4.19-4.42 (2m,2H); 7.722 (d,1H). | 70.7 |
| Sodium(5R,6S)-2- | 1.31 (d,3H); | 92 |

| | | |
|---|---|---|
| (cis-1-oxo-1-acetylimino-1-thiacyclopent-3-ylthio)-6-[(R)-1-hydroxyethyl]-2-penem-3-carboxylate (mixture of diastereomers at sulfur) | 2.15 and 2.28 (2s,3H); 2.38-2.62 (m,1H); 2.65-2.85 (m,1H); 3.53-3.85 (complex m,3H); 3.95-4.0 (2dd,1H); 4.1-4.4 (complex m,3H); 5.73 and 5.75 (2d,1H). | |
| Sodium(5R,6S)-2-(cis-1-oxo-1-methylsulfonylimino-1-thiacyclohex-4-ylthio)-6-[(R)-1-hydroxyethyl]-2-penem-3-carboxylate | 1.3 (d,3H); 2.26-2.49 (complex m,2H); 2.49-2.73 (complex m,2H); 3.21 (s,3H); 3.6-3.9 (complex m,5H); 3.95 (dd,1H); 4.18-4.32 (m,1H); 5.69 (d,1H). | 87.5 |
| Sodium(5R,6S)-2-(trans-1-oxo-1-methylsulfonylimino-1-thiacyclopent-3-ylthio)-6-[(R)-1-hydroxyethyl]-2-penem-3-carboxylate (from more polar silyl ester) | 1.3 (d,3H); 2.26-2.43 (m,1H); 2.75-2.94 (m,1H); 3.2 (s,3H); 3.48-4.4 (series m's,7H); 5.73 (d,1H). | 95 |
| Sodium(5R,6S)-2-(trans-1-oxo-1-N-methylcarbamoylimino-1-thiacyclopent-3-ylthio)-6-[(R)-1- | 1.3 (d,3H); 2.16-2.42 (m,1H); 2.68 (s,3H); 2.6-2.9 (m,1H); 3.4-3.62 (m,1H); 3.7-4.1 (complex | 74 |

| | | |
|---|---|---|
| hydroxyethyl]-2-penem-3-carboxylate (diastereomeric mixture at sulfur) | m,4H); 4.15-4.4 (m,2H); 5.716 and 5.725 (2d,1H). | |
| Sodium(5*R*,6*S*)-2-(*trans*-1-oxo-1-methoxycarbonylimino-1-thiacyclopent-3-ylthio)-6-[(R)-1-hydroxyethyl)-2-penem-3-carboxylate (from less polar silyl ester) | 1.3 (d,3H); 2.2-2.42 (m,1H); 2.72-2.98 (m,1H); 3.45-4.4 (series of m's,7H); 3.725 (s,3H); 5.716 (d,1H). | 80.7 |
| Sodium(5*R*,6*S*)-2-(*trans*-1-oxo-1-methoxycarbonylimino-1-thiacyclopent-3-ylthio)-6-[(R)-1-hydroxyethyl]-2-penem-3-carboxylate (from more polar silyl ester) | 1.3 (d,3H); 2.2-2.38 (m,1H); 2.65-2.94 (m,1H); 3.73 (d,3H); 3.42-4.4 (series complex m's,7H); 5.725 (d,1H). | 84.5 |
| Sodium(5*R*,6*S*)-2-(*cis*-1-oxo-1-imino-1-thiacyclohex-4-ylthio)-6-[(R)-1-hydroxyethyl]-2-penem-3-carboxylate | 1.3 (d,3H); 2.1-2.35 (m,2H); 2.35-2.62 (m,2H); 3.1-3.5 (complex m,4H); 3.5-3.68 (m,1H); 3.94 (dd,1H); 4.18-4.32 (m,1H); 5.677 (d,1H). | 58 |
| Sodium(5*R*,6*S*)-2-(*trans*-1-oxo-1-imino-1-thiacyclo- | 1.3 (d,3H); 2.18-2.42 (m,1H); 2.67-2.89 (m,1H); | 50 |

| | | |
|---|---|---|
| pent-3-ylthio)-6-[(R)-1-hydroxyethyl]-2-penem-3-carboxylate (mixture of dia-stereomers at sulfur | 3.21-3.56 (complex m's,3H); 3.72-3.94 (m,1H); 3.94 (2dd,1H); 4.13-4.3 (m,2H); 5.71 (d,1H). | |
| Sodium(5R,6S)-2-(cis-1-oxo-1-imino-1-thiacyclopent-3-ylthio)-6-[(R)-1-hydroxyethyl]-2-penem-3-carboxylate (from less polar silyl ester) | 1.315 (d,3H); 2.3-2.5 (m,1H); 2.66-2.88 (m,1H); 3.22-3.43 (m,2H); 3.43-3.62 (m,1H); 3.73-3.86 (m,1H); 3.97 (dd,1H); 4.05-4.35 (2m,1H); 5.73 (d,1H). | 76 |
| Sodium(5R,6S)-2-(cis-1-oxo-1-imino-1-thiacyclopent-3-ylthio)-6-[(R)-1-hydroxyethyl]-2-penem-3-carboxylate (from more polar silyl ester) | 1.316 (d,3H); 2.2-2.48 (m,1H); 2.65-2.85 (m,1H); 3.3-3.6 (m,3H); 3.7-3.95 (m,1H); 3.975 (dd,1H); 4.06-4.216 (m,1H); 4.216-4.33 (m,1H); 5.74 (d,1H). | 76 |

## Claims

**Claims for the following Contracting States : BE, CH, DE, FR, GB, IT, LI, LU, NL, SE**

1. A compound of the formula

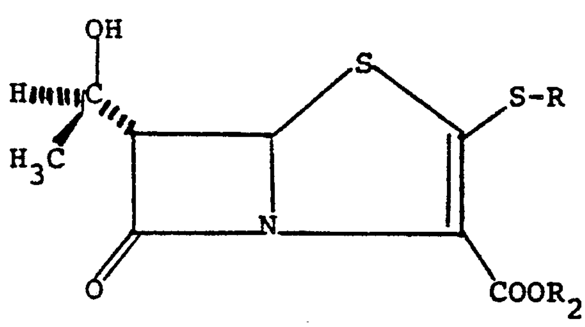

(I)

or a pharmaceutically acceptable salt thereof wherein R is a 4, 5 or 6 membered thiacycloalkane moiety of the formula

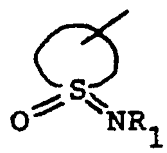

wherein $R_1$ is hydrogen, $C_1$ to $C_4$ alkoxycarbonyl, $C_1$ to $C_4$ alkylcarbonyl, $C_1$ to $C_4$ alkylsulfonyl, $C_1$ to $C_4$ alkylaminocarbonyl, and $R_2$ is hydrogen or an ester-forming group hydrolyzable in vivo.

2. A compound according to claim 1 wherein R is cis-1-oxo-1-imino-1-thiacyclopent-3-yl and $R_2$ is hydrogen.

3. A compound according to claim 1 wherein R is trans-1-oxo-1-imino-1-thiacyclopent-3-yl and $R_2$ is hydrogen.

4. A compound according to claim 1 wherein R is cis-1-oxo-1-imino-thiacyclohex-4-yl and $R_2$ is hydrogen.

5. A compound according to claim 1 wherein R is trans-1-oxo-1-imino-thiacyclohex-4-yl and $R_2$ is hydrogen.

6. An antibacterial pharmaceutical composition comprising a compound according to claim 1 and a pharmaceutically acceptable carrier or diluent.

7. A compound of the formula I as claimed in any one of claims 1 to 5 for use in medicine as an antibacterial agent.

**Claims for the following Contracting States : AT, ES, GR**

1. A process for the preparation of a compound of the formula

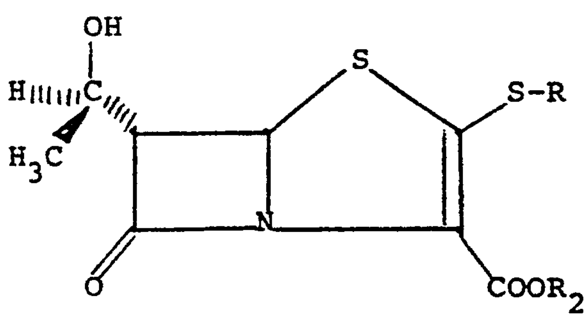

(I)

or a pharmaceutically acceptable salt thereof wherein R is a 4, 5 or 6 membered thiacycloalkane moiety of the formula

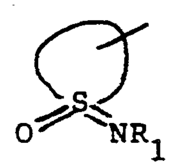

wherein $R_1$ is hydrogen, $C_1$-$C_4$ alkoxycarbonyl, $C_1$-$C_4$ alkylcarbonyl, $C_1$-$C_4$ alkylsulfonyl, $C_1$-$C_4$ alkylaminocarbonyl, and $R_2$ is hydrogen or an ester-forming group hydrolyzable in vivo comprising the step of hydrogenating a compound of Formula (I) wherein $R_2$ is a carboxylic acid protecting group.

2. A process according to claim 1 wherein R is cis-1-oxo-1-imino-1-thiacyclopent-3-yl and $R_2$ is hydrogen.

3. A process according to claim 1 wherein R is trans-1-oxo-1-imino-1-thiacyclopent-3-yl and $R_2$ is hydrogen.

4. A process according to claim 1 wherein R is cis-1-oxo-1-imino-thiacyclohex-4-yl and $R_2$ is hydrogen.

5. A process according to claim 1 wherein R is trans-1-oxo-1-imino-thiacyclohex-4-yl and $R_2$ is hydrogen.

**Revendications**

**Revendications pour les Etats contractants suivants : BE, CH, DE, FR, GB, IT, LI, LU, NL, SE**

1. Composé de formule

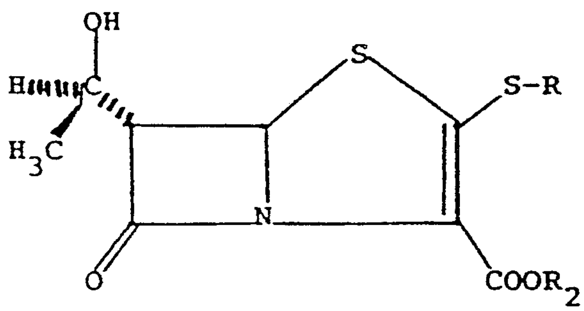

(I)

ou un sel pharmaceutiquement acceptable de ce composé, formule dans laquelle R est un groupement thiacycloalcane à 4, 5 ou 6 chaînons de formule

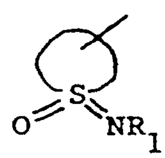

dans laquelle $R_1$ est l'hydrogène, un groupe (alkoxy en $C_1$ à $C_4$)carbonyle, (alkyle en $C_1$ à $C_4$)-carbonyle, alkylsulfonyle en $C_1$ à $C_4$, (alkyle en $C_1$ à $C_4$)aminocarbonyle, et $R_2$ est l'hydrogène ou un groupe formant un ester, hydrolysable in vivo.

2. Composé suivant la revendication 1, dans lequel R est le groupe cis-1-oxo-1-imino-1-thiacyclopent-3-yle et $R_2$ est l'hydrogène.

3. Composé suivant la revendication 1, dans lequel R est le groupe trans-1-oxo-1-imino-1-thiacyclopent-3-yle et $R_2$ est l'hydrogène.

4. Composé suivant la revendication 1, dans lequel R est le groupe cis-1-oxo-1-imino-thiacyclohex-4-yle et $R_2$ est l'hydrogène.

5. Composé suivant la revendication 1, dans lequel R est le groupe trans-1-oxo-1-imino-thiacyclohex-4-yle et $R_2$ est l'hydrogène.

6. Composition pharmaceutique antibactérienne, comprenant un composé suivant la revendication 1 et un support ou diluant acceptable du point de vue pharmaceutique

7. Composé de formule I suivant l'une quelconque des revendications 1 à 5, destiné à être utilisé en médecine comme agent antibactérien.

**Revendications pour les Etats contractants suivants : AT, ES, GR**

1. Procédé de préparation d'un composé de formule :

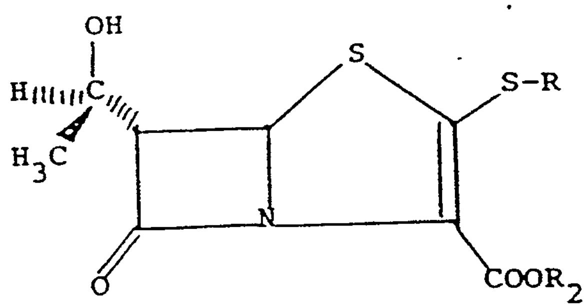

(I)

ou d'un sel pharmaceutiquement acceptable de ce composé, formule dans laquelle R est un groupement thiacycloalcane à 4, 5 ou 6 chaînons de formule

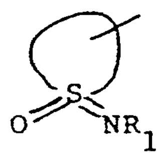

dans laquelle $R_1$ est l'hydrogène, un groupe (alkoxy en $C_1$ à $C_4$)carbonyle, (alkyle en $C_1$ à $C_4$)-carbonyle, alkylsulfonyle en $C_1$ à $C_4$, (alkyle en $C_1$ à $C_4$)aminocarbonyle, et $R_2$ est l'hydrogène ou un groupe formant un ester, hydrolysable in vivo, comprenant l'étape d'hydrogénation d'un composé de formule (I) dans laquelle $R_2$ est un groupe protégeant la fonction acide carboxylique.

2. Procédé suivant la revendication 1, dans lequel R est le groupe cis-1-oxo-1-imino-1-thiacyclopent-3-yle et $R_2$ est l'hydrogène.

3. Procédé suivant la revendication 1, dans lequel R est le groupe trans-1-oxo-1-imino-1-thiacyclopent-3-yle et $R_2$ est l'hydrogène.

4. Procédé suivant la revendication 1, dans lequel R est le groupe cis-1-oxo-1-imino-thiacyclohex-4-yle et $R_2$ est l'hydrogène.

5. Procédé suivant la revendication 1, dans lequel R est le groupe trans-1-oxo-1-imino-thiacyclohex-4-yle et $R_2$ est l'hydrogène.

**Patentansprüche**

**Patentansprüche für folgende Vertragsstaaten : BE, CH, DE, FR, GB, IT, LI, LU, NL, SE**

1. Verbindung der Formel

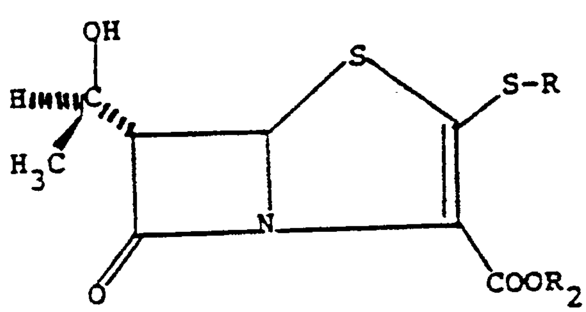

(I)

oder ein pharmazeutisch verwendbares Salz davon, worin R einen 4-, 5- oder 6-gliedrigen Thiacycloalkan-Rest der Formel

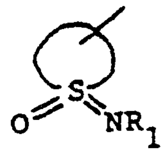

darstellt, worin $R_1$ Wasserstoff, $C_1$-$C_4$-Alkoxycarbonyl, $C_1$-$C_4$-Alkylcarbonyl, $C_1$-$C_4$-Alkylsulfonyl oder $C_1$-$C_4$-Alkylaminocarbonyl und $R_2$ Wasserstoff oder eine Ester-bildende, in vivo hydrolysierbare Gruppe bedeuten.

2. Verbindung nach Anspruch 1, worin R cis-1-Oxo-1-imino-1-thiacyclopent-3-yl und $R_2$ Wasserstoff sind.

3. Verbindung nach Anspruch 1, worin R trans-1-Oxo-1-imino-1-thiacyclopent-3-yl und $R_2$ Wasserstoff sind.

4. Verbindung nach Anspruch 1, worin R cis-1-Oxo-1-imino-thiacyclohex-4-ylund $R_2$ Wasserstoff sind.

5. Verbindung nach Anspruch 1, worin R trans-1-Oxo-1-imino-thiacyclohex-4-yl und $R_2$ Wasserstoff sind.

6. Antibakterielle pharmazeutische Zusammensetzung, enthaltend eine Verbindung nach Anspruch 1 und einen Träger oder ein Verdünnungsmittel, die pharmazeutisch verwendbar sind.

7. Verbindung der Formel I, wie beansprucht in einem der Ansprüche 1 bis 5, zur Verwendung als antibakterielles Mittel in der Medizin.

**Patentansprüche für folgende Vertragsstaaten : AT, ES, GR**

1. Verfahren zur Herstellung einer Verbindung der Formel

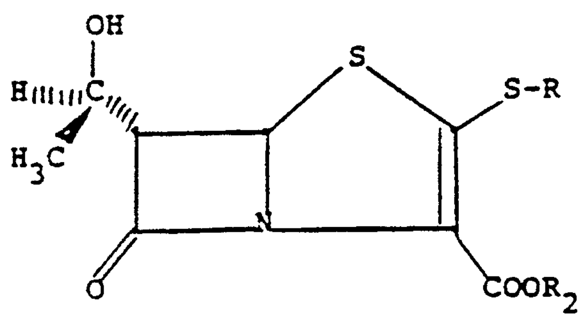

(I)

oder eines pharmazeutisch verwendbaren Salzes davon, worin R einen 4-, 5- oder 6-gliedrigen Thiacycloalkan-Rest der Formel

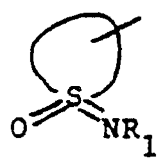

darstellt, worin $R_1$ Wasserstoff, $C_1$-$C_4$-Alkoxycarbonyl, $C_1$-$C_4$-Alkylcarbonyl, $C_1$-$C_4$-Alkylsulfonyl oder $C_1$-$C_4$-Alkylaminocarbonyl und $R_2$ Wasserstoff oder eine Ester-bildende, in vivo hydrolysierbare Gruppe bedeuten, enthaltend die Stufe der Hydrierung einer Verbindung der Formel (I), worin $R_2$ eine Schutzgruppe für eine Carbonsäure ist.

2. Verfahren nach Anspruch 1, worin R cis-1-Oxo-1-imino-1-thiacyclopent-3-yl und $R_2$ Wasserstoff sind.

3. Verfahren nach Anspruch 1, worin R trans-1-Oxo-1-imino-1-thiacyclopent-3-yl und $R_2$ Wasserstoff sind.

4. Verfahren nach Anspruch 1, worin R cis-1-Oxo-1-imino-thiacyclohex-4-yl und $R_2$ Wasserstoff sind.

5. Verfahren nach Anspruch 1, worin R trans-1-Oxo-1-imino-thiacyclohex-4-yl und $R_2$ Wasserstoff sind.